(19) Europäisches Patentamt
European Patent Office
Office européen des brevets

(11) Numéro de publication : **0 120 770**
**B1**

(12) # FASCICULE DE BREVET EUROPÉEN

(45) Date de publication du fascicule du brevet : **23.03.88**

(21) Numéro de dépôt : **84400548.8**

(22) Date de dépôt : **19.03.84**

(51) Int. Cl.[4] : **C 07 D213/38**, C 07 D241/12, C 07 D401/12, A 61 K 31/44, A 61 K 31/50

(54) **Dérivés de 2-aminoéthyl pyridine ou pyrazine, leur préparation et les compositions pharmaceutiques les contenant.**

(30) Priorité : **22.03.83 FR 8304690**

(43) Date de publication de la demande :
**03.10.84 Bulletin 84/40**

(45) Mention de la délivrance du brevet :
**23.03.88 Bulletin 88/12**

(84) Etats contractants désignés :
**AT BE CH DE FR GB IT LI LU NL SE**

(56) Documents cités :
**AT-B- 344 180**
**FR-A- 1 385 772**
**FR-M- 5 390**
**GB-A- 1 551 993**
**US-A- 3 952 101**

(73) Titulaire : **RIOM LABORATOIRES- C.E.R.M. "R.L.-CERM" - (S.A.)**
**Route de Marsat B.P. 140**
**F-63203 Riom Cedex (FR)**

(72) Inventeur : **Simond, Jacques Aimé Louis**
**Rue des Thuilets Mirefleurs**
**F-63730 Les Martes de Veyre (FR)**
Inventeur : **Carlier, Patrick**
**La Gravière 10 Fonfreyde**
**F-63140 Châtel-Guyon (FR)**
Inventeur : **Monteil, André Jean-Claude**
**Route de Prompsat Les grosliers**
**F-63140 Châtel-Guyon (FR)**

(74) Mandataire : **Thérond, Gérard Raymond et al**
**RIOM LABORATOIRES - CERM - S.A. Route de Marsat**
**B.P. 140**
**F-63203 Riom Cedex (FR)**

**EP 0 120 770 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux dérivés d'aminoéthyl pyridine ou pyrazine ainsi que leurs méthodes de préparation.

L'invention a plus particulièrement pour objet des dérivés de (1-alcoxy-2-amino) pyridine ou pyrazine de formule générale I :

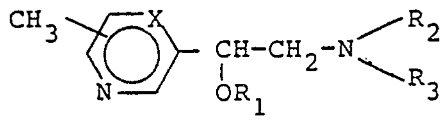

(I)

et leurs sels pharmaceutiquement acceptables, dans laquelle X représente soit -CH= soit -N=, $R_1$ représente un radical alkyle ayant 1 à 5 atomes de carbone, $R_2$ représente l'hydrogène et $R_3$ est un radical alkyle ayant 1 à 5 atomes de carbone ou bien $R_2$ et $R_3$ représentent ensemble avec l'atome d'azote auquel ils sont liés un reste pyrrolidinyle, morpholino, 4-phényl pipérazinyle dans lequel le radical phényle peut porter un ou plusieurs des substituants suivants un halogène, un radical alkyle ou alcoxy ayant 1 à 5 atomes de carbone, ou le radical trifluorométhyle, ou 4-(2-pyrimidinyl)-pipérazinyle.

L'invention concerne également les compositions pharmaceutiques contenant les composés de formule (I), et leurs sels pharmaceutiquement acceptables, notamment en raison de leurs intéressantes propriétés anti-bronchoconstrictrices.

On connaît déjà (par les brevets français n° 1 385 772 et n° 1 404 442) des composés présentant un enchaînement 2-alcoxy éthylamino, correspondant à la formule générale II :

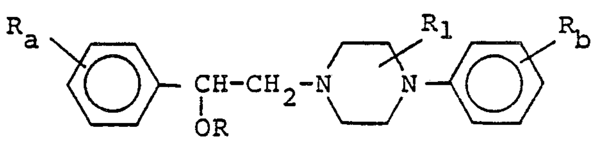

(II)

dans laquelle $R_a$ est un halogène ; $R_b$ est un atome d'hydrogène, un radical alkyle ou alcoxy inférieur ou un halogène ; R est un radical alkyle inférieur et $R_1$ représente H ou $CH_3$. Ces composés sont divulgués comme ayant des propriétés anti-inflammatoires, hypotensives, diurétiques ou sali-diurétiques.

On connaît également (par le brevet anglais n° 1 551 993 et le brevet autrichien n° 344 180) des composés dans lesquels le radical phényle (non lié à l'azote) est remplacé par un radical hétérocyclique non substitué. Selon la réalisation la plus proche des composés de l'invention, ces composés répondent à la formule générale III :

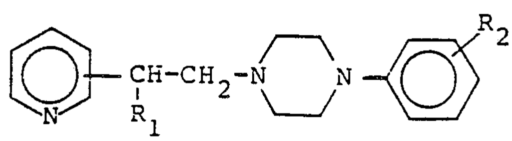

(III)

dans laquelle $R_1$ représente H, OH ou un radical alcoxy inférieur ; $R_2$ représente H, un radical alkyle ou alcoxy inférieur ou un halogène. Lesdits composé sont divulgués comme possédant des propriétés antihypertensives, antihistaminiques et antibradykinines.

Le brevet US n° 3 952 101 décrit également pour leurs propriétés antibronchoconstrictrices des aminoéthylpyridines, différentes des composés de l'invention, notamment en ce qu'elles comportent deux substitutions hydroxy.

Par rapport à ces familles de produits, les composés de l'invention présentent des propriétés soit différentes, soit d'intensité très supérieure conduisant à un index thérapeutique beaucoup plus favorable.

Par rapport aux composés de structure III, les composés de l'invention de formule I sont caractérisés par le fait que l'atome d'azote de l'hétérocycle est toujours en position 3 par rapport au carbone de liaison et que ledit hétérocycle est toujours substitué par un radical méthyle. De préférence, dans les composés selon l'invention, X représente -CH=, le radical méthyle est en position 4, et

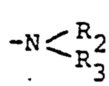

représente un radical phényl pipérazinyle éventuellement substitué sur le phényle.

Les composés de l'invention peuvent être préparés selon les procédés connus pour la préparation de composés analogues.

Une méthode générale pour préparer lesdits composés consiste à condenser un composé de formule IV :

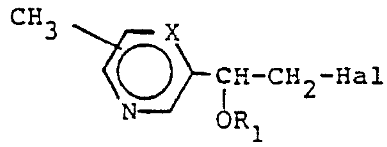

(IV)

dans laquelle X et $R_1$ ont les significations précédemment indiquées et « Hal » désigne un atome d'halogène, de préférence de chlore ou le brome, avec une amine de formule V :

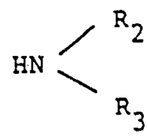

(V)

ou un dérivé actif de cette amine dans laquelle l'hydrogène a été remplacé par un métal ou un dérivé métallique, tel que le sodium ou le lithium, dans laquelle $R_2$ et $R_3$ ont les significations précédemment indiquées.

Selon une réalisation préférée, les composés de l'invention sont préparés en deux stades à partir d'une 3-vinyl pyridine ou 2-vinyl pyrazine substituée par un radical méthyle, conformément au schéma réactionnel suivant :

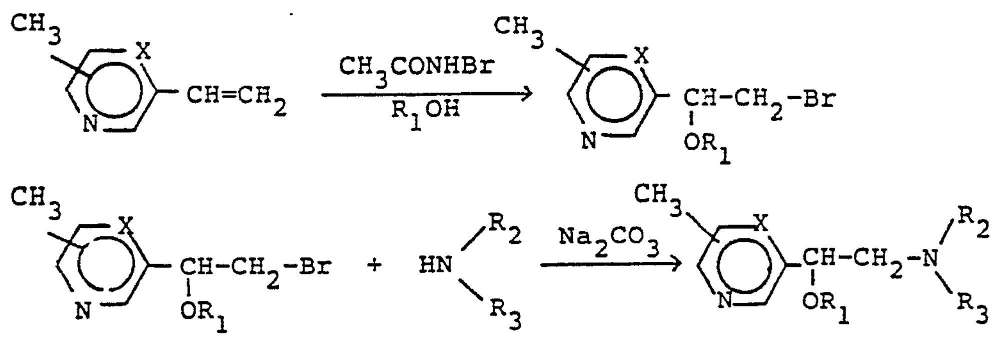


Dans la première étape, on soumet une vinyl pyridine ou pyrazine à une alkoxy bromuration par un N-bromo amide ou N-bromo imide en présence d'un alcool $R_1OH$ correspondant à la définition de $R_1$.

Dans la deuxième étape, on condense le dérivé obtenu avec une amine (V)$NHR_2R_3$, en présence d'un solvant organique et de carbonate de sodium par chauffage à reflux.

Les sels pharmaceutiquement acceptables des composés de formule I sont les sels d'addition acide obtenus en faisant agir la base libre avec un acide organique ou minéral pharmaceutiquement acceptable, tel que HCl, HBr, l'acide acétique, l'acide phosphorique, l'acide méthanesulfonique, l'acide maléïque, l'acide fumarique, l'acide succinique, l'acide tartrique, etc...

Dans la définition de $R_1$ et $R_2$ le terme alkyle désigne un radical alkyle ayant 1 à 5 atomes de carbone tel que méthyle, éthyle, propyle, ter-butyl, sec-butyl, pentyl.

L'activité des composés de l'invention en thérapeutique, notamment en tant qu'agents antibronchoconstricteurs a été mise en évidence à l'issue de tests pharmacologiques dont les protocoles sont résumés ci-après.

L'activité antibronchoconstrictrice a été recherchée chez le cobaye tricolore selon la technique de Konzett et Rossler. Les animaux sont anesthésiés au carbamate d'éthyle et après trachéotomie sont ventilés artificiellement à volume constant ; on enregistre à chaque instant la pression intratrachéale. L'histamine (5 $\mu g. kg^{-1}$ I.V.) est administrée avant et à différentes intervalles de temps après traitement par la substance à l'étude. L'activité du produit administré par voie I.V., est évaluée en comparant les amplitudes des bronchospasmes avant et après traitement et en calculant le pourcentage de variation et la durée d'action.

Les résultats sont rapportés dans le tableau I.

(Voir Tableau I p. 4)

Tableau I

| COMPOSE N° ˣ | DOSE (mg I.V.) | % D'INHIBITION DU BRONCHOSPASME A L'HISTAMINE | DUREE en min. |
|---|---|---|---|
| 1 | 5 | 56,5 | > 60 |
| 2 | 0,05 | 85 | > 120 |
| 3 | 5 | 50 | > 15 |
| 6 | 0,025 | 90 | > 120 |
| 7 | 0,25 | 97 | > 60 |
| 8 | 0,1 | 95 | > 15 |
| 9 | 0,25 | 98 | 30 |
| 10 | 1 | 100 | > 60 |
| 11 | 0,25 | 100 | > 60 |
| 12 | 0,5 | 91 | > 60 |
| 14 | 5 | 82 | 45 |
| 15 | 0,05 | 80 | 60 |
| 16 | 0,1 | 79 | 45 |

ˣ Voir tableau II.

Ces résultats font apparaître que les composés de l'invention possèdent d'intéressantes propriétés antibronchoconstrictrices.

On notera en particulier l'activité des composés n° 2, 6, 7, 8, 15, 16 et surtout celle du composé n° 6, particulièrement remarquable, tant en intensité qu'en durée d'action. Pour ce dernier composé la valeur de l'ED 50 dans ce test est de 0,0019 $mg.kg^{-1}$.

Cette activité antibronchoconstrictrice est également confirmée par d'autres tests pharmacologiques classiques dans cette indication.

Les essais pharmacologiques ont également montré que les composés de l'invention ne présentaient pas de toxicité élevée (La DL 50 P.O. chez la souris est comprise entre 300 et 800 $mg.kg^{-1}$).

Les composés de formule I et leurs sels pharmaceutiquement acceptables peuvent donc être utilisés par exemple pour le traitement des bronchites spastiques, des insuffisances respiratoires chroniques, des allergies respiratoires et pour les explorations fonctionnelles pulmonaires.

En association avec les excipients pharmaceutiques habituels, ils pourront être utilisés en thérapeutique humaine à des doses quotidiennes comprises entre 1 et 100 mg, de préférence entre 10 et 100 mg selon les modes d'administration.

Les modes préférés d'administration sont l'administration par voie orale ou par injection.

Bien entendu, le véhicule ou excipient doit être choisi en fonction de la voie retenue pour l'administration.

La présentation galénique des compositions de l'invention n'offre pas de difficultés et fait appel à des moyens connus de l'homme de métier. A titre purement illustratif, on a présenté ci-dessous deux exemples de réalisation galénique.

Une composition appropriée pour les comprimés est la suivante :

| | |
|---|---|
| Composé n° 6 | 4 mg |
| Lactose | 80 mg |
| Amidon de blé | 12,5 mg |
| Polyvidone K 30 | 3 mg |
| Stéarate de magnésium | 0,5 mg |

Pour réaliser un lot de 1 000 comprimés, on mélange les quantités voulues de composé n° 6, de lactose et d'amidon de blé, puis on mouille avec une solution de polyvidone.

Lorsque le mélange est homogène, on extrude le granulé, on le sèche, on ajoute le stéarate de magnésium, puis on comprime pour obtenir des comprimés d'un poids moyen de 100 mg.

Une composition injectable est par exemple la suivante :

| | |
|---|---|
| Composé n° 6 | 3 mg |
| Propane diol 1,2 | 1,5 ml |
| Glucose | 250 mg |
| Eau PPI q.s. | 5 ml |

Pour préparer un lot de 2 litres, en utilisant les proportions de la formule centésimale, on dissout le

composé n° 6 dans le propane diol 1,2 et le glucose dans une partie de l'eau, puis on réunit les deux solutions et on complète le volume à 2 litres.

La solution ainsi obtenue est ensuite filtrée sur une membrane d'acétate de cellulose 0,22 μm et on répartit en ampoules de 5,15 ml.

Les exemples ci-après illustrent la préparation des composés de l'invention de formule I :

Exemple 1 : 1-[2-méthoxy-2-(6-méthyl-3-pyridyl)-éthyl]-4-phényl pipérazine

1. Dans un ballon contenant 1 litre de méthanol, on a ajouté progressivement, sous agitation, 154,5 g de N-bromoacétamide en maintenant la température à 0 °C, puis 133,2 g de 2-méthyl 5-vinyl pyridine en maintenant l'agitation jusqu'à ce que la température revienne à la température ambiante.

Lorsque la réaction était terminée, on a distillé sous vide la 2-méthyl 5-(1-méthoxy 2-bromo) éthyl pyridine. L'huile obtenue a ensuite été purifiée par les moyens habituels, puis redistillée sur carbonate de sodium. On a obtenu 150 g de produit ayant pour point d'ébullition $E_{0,5}$ = 94 °C.

2. Dans un ballon muni d'un réfrigérant, on a introduit 16,1 g (0,07 M) de 2-méthyl 5-(1-méthoxy 2-bromo) éthyl pyridine et 9,4 g de N-phényl pipérazine en solution dans 50 ml de butanol.

On a ajouté 9,4 g de carbonate de sodium et on a porté le mélange à reflux pendant 30 heures. La fin de la réaction était contrôlée par chromatographie sur couche mince. Après élimination du carbonate de sodium par filtration, on a laissé cristalliser le produit du titre dans l'éthanol. Après recristallisation dans l'acétate d'éthyle, on a obtenu 7,2 g de produit du titre ayant pour point de fusion F = 93 °C.

Exemple 2 : 1-[2-méthoxy-2-(6-méthyl-3-pyridyl)-éthyl]-4-(2-méthoxy-phényl) pipérazine

En procédant comme indiqué dans l'exemple 1, on a préparé la 2-méthyl-5-(1-méthoxy 2-bromo) éthyl pyridine qu'on a ensuite fait réagir avec la 2-méthoxy phényl pipérazine dans le butanol comme solvant, en présence de carbonate de sodium. En partant de 16,1 g (0,07 M) de dérivé bromé et de 11,1 g (0,058 M) de 2-méthoxy phényl pipérazine dans 50 ml de butanol en présence de 12 g de carbonate de sodium, on a obtenu, après chauffage à reflux pendant 21 heures, 10 g de produit du titre ayant pour point de fusion F = 88 °C.

On a également transformé la base en sel en faisant agir l'acide fumarique ; on a obtenu un produit cristallisé ayant pour point de fusion F = 153 °C.

Exemple 3 : 1-[2-isobutoxy-2-(6-méthyl-3-pyridyl)-éthyl]-4-(2-méthoxy phényl) pipérazine

a. Dans un ballon contenant 300 ml d'isobutanol, on a ajouté progressivement sous agitation 41,4 g de N-bromoacétamide en maintenant la température à 0 °C, puis 35,7 g de 2-méthyl-5-vinyl pyridine en maintenant l'agitation jusqu'à ce que la température revienne à température ambiante.

On a ensuite laissé le milieu réactionnel à température ambiante pendant 48 heures, puis on a distillé le dérivé bromé formé. On a obtenu 87,4 g de produit qu'on a utilisé au stade suivant.

b. On a fait réagir 23,6 g (0,1 M) du dérivé bromé précédent et 15 g (0,08 M) de 2-méthoxy phényl pipérazine dans 100 ml de butanol en présence de 15 g de carbonate de sodium, en maintenant le mélange réactionnel à reflux pendant 10 heures, en suivant la fin de réaction par chromatographie sur couche mince. Après élimination du carbonate par filtration et évaporation à sec du butanol, on a ajouté de l'acide fumarique pour obtenir 2,2 g de difumarate du composé du titre ayant pour point de fusion F = 144 °C.

Exemple 4 : N-[2-méthoxy-2-(6-méthyl-3-pyridyl)] éthyl-N-terbutylamine

En partant de 23 g de dérivé bromé approprié et de 21,9 g de terbutylamine dans 200 ml de butanol en présence de 50 g de carbonate de potassium, puis en traitant par l'acide fumarique, on a obtenu le composé du titre sous forme de fumarate (1 ; 1,5) ayant pour point de fusion F = 221,6 °C.

Exemple 5 : 1-[2-méthoxy-2-(3-méthyl-2-pyrazinyl) éthyl]-4-phényl pipérazine

A un mélange de 90 ml de méthanol et de 11 g de 2-méthyl 3-vinyl pyrazine, on a ajouté 12,7 g de N-bromoacétamide en maintenant la température entre 0 °C et -5 °C, puis on a laissé le milieu réactionnel revenir à température ambiante. On a extrait et purifié le dérivé bromé et obtenu 10 g de produit ayant pour point d'ébullition $E_{0,4}$ = 99 °C.

Dans le deuxième stade, on a condensé la 2-méthyl-3-(1-méthoxy-2-bromo) éthyl pyrazine et la phényl pipérazine.

En partant de 10 g de dérivé bromé, de 7 g de phényl pipérazine, de 100 ml de butanol et de 10 g de carbonate de potassium, on a obtenu 10 g de composé du titre qu'on a cristallisé sous forme de fumarate ayant pour point de fusion F = 157 °C.

En utilisant le même procédé, on a également préparé les composés dont les caractéristiques sont résumées dans le tableau II :

Tableau II

| COMPOSE N° | 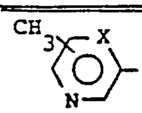 | $R_1$ | 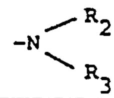 | SEL POINT FUSION |
|---|---|---|---|---|
| 1 | 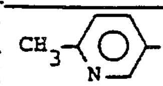 | $-CH_3$ | 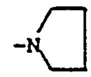 | Fumarate 159°C |
| 2 | 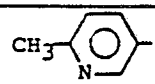 | $-CH_3$ | 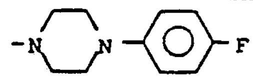 | Trichl$^{te}$ 234°C |
| 3 | 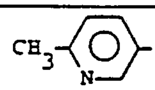 | $-CH_3$ | 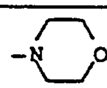 | Fumarate 152°C |
| 4 (Ex. 3) | 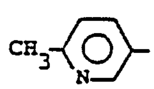 | 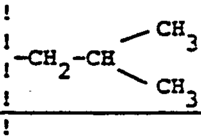 | 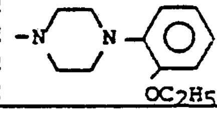 | Difumarate 144°C |
| 5 | 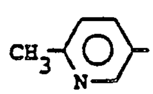 | $-CH_3$ | 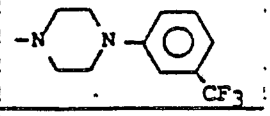 | Difumarate 155°C |
| 6 (Ex. 1) | 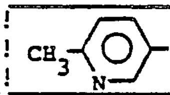 | $-CH_3$ | 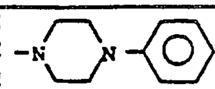 | Base 93°C |
| 7 (Ex. 2) | 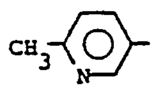 | $-CH_3$ | 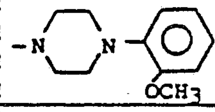 | Base 88°C |
| 8 | 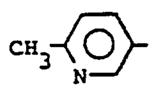 | $-CH_3$ | 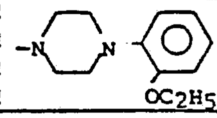 | Base 71°C |
| 9 | 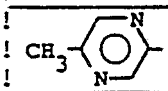 | $-CH_3$ | 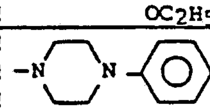 | Base 63°C |
| 10 | 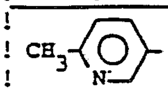 | $-C_2H_5$ | 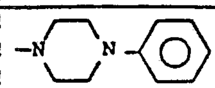 | Trichl$^{te}$ 201,5°C |
| 11 | 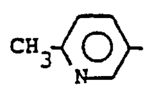 | $-C_2H_5$ | 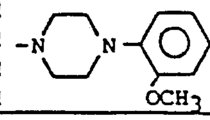 | Base 73°C |
| 12 | 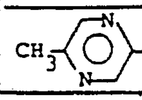 | $-CH_3$ | 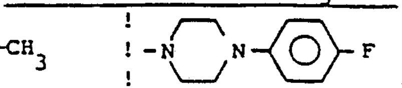 | Base 79,5°C |
| 13 (Ex. 4) | 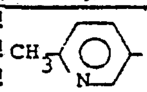 | $-CH_3$ | 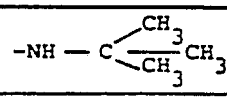 | 1,5 Fumarate 221,6°C |
| 14 (Ex. 5) | 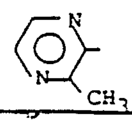 | $-CH_3$ | 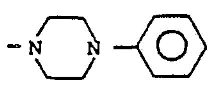 | Fumarate 157°C |
| 15 | 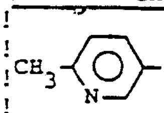 | $-CH_3$ | 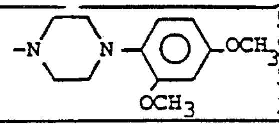 | Trichl$^{te}$ 226°C |
| 16 | 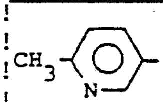 | $-CH_3$ | 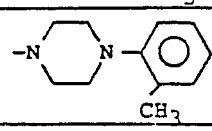 | Trichl$^{te}$ 204°C |

Tableau II (Suite)

| COMPOSE N° | 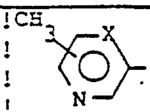 | $R_1$ | 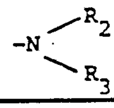 | SEL POINT FUSION |
|---|---|---|---|---|
| 17 | 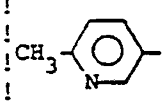 | $-CH_3$ | 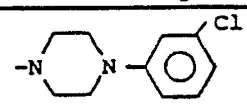 | Base liquide $E_{0,1}$ = 220°C |
| 18 | 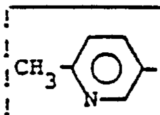 | $-CH_3$ | 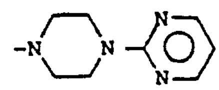 | Base liquide $E_{0,1}$ = 190°C |
| 19 | 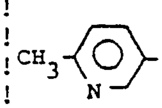 | $-CH_3$ | 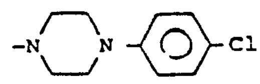 | Base liquide $E_{0,1}$ = 200°C |

**Revendications**

1. Composés chimiques, caractérisés en ce qu'ils répondent à la formule :

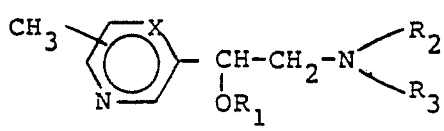

et leurs sels pharmaceutiquement acceptables dans laquelle X représente soit -CH=, soit -N=, $R_1$ représente un radical alkyle ayant 1 à 5 atomes de carbone, $R_2$ représente l'hydrogène et $R_3$ est un radical alkyle ayant 1 à 5 atomes de carbone ou bien $R_2$ et $R_3$ représentent ensemble avec l'atome d'azote auquel ils sont liés, un reste pyrrolidinyle, morpholino, 4-phényl pipérazinyle dans lequel le radical phényle peut porter un ou plusieurs des substituants suivants un halogène, un radical alkyle ou alcoxy ayant 1 à 5 atomes de carbone, ou le radical trifluorométhyle, ou 4-(2-pyrimidinyl)-pipérazinyle.

2. Composés selon la revendication 1 caractérisés en ce que X représente -CH= et que le radical méthyle est fixé sur le radical pyridyle en position 4 par rapport au carbone de liaison.

3. Composé selon la revendication 1, caractérisé en ce que $R_1$ représente le radical méthyle et $-NR_2R_3$ le radical 4-phényl pipérazinyle.

4. Composé selon la revendication 1, caractérisé en ce que $R_1$ représente le radical méthyle et $-NR_2R_3$ le radical 4-(2-méthoxy) phényl pipérazinyle.

5. Procédé pour la préparation des composés selon la revendication 1 caractérisé en ce qu'on fait réagir un composé de formule :

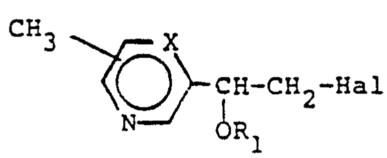

dans laquelle X et $R_1$ ont la signification indiquée dans la revendication 1 et Hal désigne un halogène, avec une amine de formule :

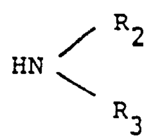

dans laquelle $R_2$ et $R_3$ ont la signification précédemment indiquée, ou dérivé actif de ladite amine dans laquelle l'hydrogène a été remplacé par un métal ou un dérivé métallique.

6. Composition pharmaceutique caractérisée en ce qu'elle contient, à titre de principe actif, un composé selon la revendication 1.

**Claims**

1. Chemical compounds characterized in that they correspond to the formula :

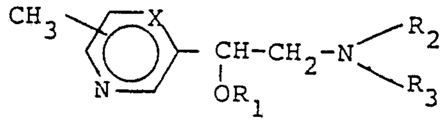

and pharmaceutically acceptable salts thereof, in which X represents either -CH = or -N =, $R_1$ represents an alkyl radical having 1 to 5 carbon atoms, $R_2$ represents hydrogen and $R_3$ is an alkyl radical having 1 to 5 carbon atoms, or $R_2$ and $R_3$ together with the nitrogen atom to which they are bonded represent pyrrolidinyl or morpholino residue or a 4-phenylpiperazinyl residue in which the phenyl radical may carry one or more of the following substituents, namely a halogen, an alkyl or alkoxy radical having 1 to 5 carbon atoms or a trifluoromethyl radical; or a 4-(2-pyrimidinyl)piperazinyl residue.

2. Compounds according to Claim 1, characterized in that X represents -CH =, and in that the methyl radical is attached to the pyridyl radical in the 4-position relative to the bonding carbon.

3. Compound according to Claim 1, characterized in that $R_1$ represents a methyl radical and $-NR_2R_3$ a 4-phenylpiperazinyl radical.

4. Compound according to Claim 1, characterized in that $R_1$ represents a methyl radical and $-NR_2R_3$ a 4-(2-methoxyphenyl)piperazinyl radical.

5. Process for the preparation of the compounds according to Claim 1, characterized in that a compound of formula :

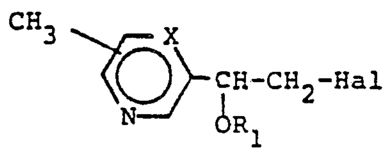

in which X and $R_1$ have the meanings assigned in Claim 1 and Hal denotes a halogen, is reacted with an amine of formula :

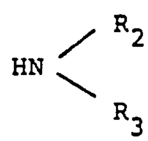

in which $R_2$ and $R_3$ have the meaning assigned above, or an active derivative of the said amine in which the hydrogen has been replaced by a metal or a metal derivative.

6. Pharmaceutical composition, characterized in that it contains, by way of an active principale, a compound according to Claim 1.

**Patentansprüche**

1. Chemische Verbindungen, dadurch gekennzeichnet, dass sie der Formel :

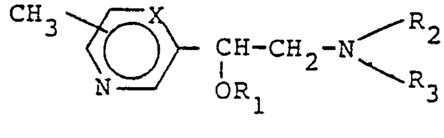

und deren pharmazeutisch unbedenklichen Salzen entsprechen, worin X entweder für -CH = oder -N =, $R_1$ für einen Alkylrest mit 1 bis 5 Kohlenstoffatomen, $R_2$ für Wasserstoff und $R_3$ für einen Alkylrest mit 1 bis 5 Kohlenstoffatomen stehen oder auch $R_2$ und $R_3$ zusammen mit dem Stickstoffatom, an das sie gebunden sind, einen Pyrrolidinyl-, Morpholino-, 4-Phenylpiperazinyl-, in welchem der Phenylrest einen oder mehrere der folgenden Substituenten, und zwar Halogen, einen Alkyl- oder Alkoxyrest mit 1 bis 5 Kohlenstoffatomen oder den Trifluormethylrest tragen kann, oder 4-(2-Pyrimidinyl)-piperazinylrest darstellen.

2. Verbindungen nach Anspruch 1, dadurch gekennzeichnet, dass X für -CH = steht und der Methylrest am Pyridylrest in 4-Stellung zur Kohlenstoffbindung steht.

3. Verbindung nach Anspruch 1, dadurch gekennzeichnet, dass $R_1$ für den Methylrest und $-NR_2R_3$ für den 4-Phenylpiperazinylrest stehen.

4. Verbindung nach Anspruch 1, dadurch gekennzeichnet, dass $R_1$ für den Methylrest und $-NR_2R_3$ für den 4-(2-Methoxy)-phenylpiperazinylrest stehen.

5. Verfahren zur Herstellung der Verbindungen nach Anspruch 1, dadurch gekennzeichnet, dass man eine Verbindung der Formel :

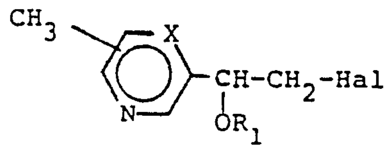

worin X und $R_1$ die in Anspruch 1 angegebene Bedeutung haben und Hal ein Halogen bezeichnet, mit einem Amin der Formel :

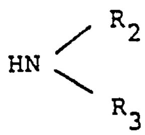

worin $R_2$ und $R_3$ die zuvor angegebenen Bedeutungen haben, oder einem aktiven Derivat des besagten Amins, worin der Wasserstoff durch ein Metall oder Metallderivat ersetzt ist, umsetzt.

6. Pharmazeutische Zusammensetzung, dadurch gekennzeichnet, dass sie als Wirkstoff eine Verbindung nach Anspruch 1 enthält.